Europäisches Patentamt

European Patent Office

Office européen des brevets

(11) Numéro de publication: **0 389 332**
**A1**

## (12) DEMANDE DE BREVET EUROPEEN

(21) Numéro de dépôt: 90400660.8

(22) Date de dépôt: **13.03.90**

(51) Int. Cl.5: **A61B 6/04**

(30) Priorité: **23.03.89 FR 8903846**

(43) Date de publication de la demande:
**26.09.90 Bulletin 90/39**

(84) Etats contractants désignés:
**DE ES GB IT NL**

(71) Demandeur: **GENERAL ELECTRIC CGR S.A.**
**100, rue Camille-Desmoulins**
**F-92130 Issy les Moulineaux(FR)**

(72) Inventeur: **Jarin, Jean-Pierre**
**Cabinet Ballot-Schmit**
**7, rue Le Sueur, F-75116 Paris(FR)**

(74) Mandataire: **Ballot, Paul Denis Jacques et al**
**Cabinet Ballot-Schmit 7, rue le Sueur**
**F-75116 Paris(FR)**

(54) **Support patient à grand débattement vertical.**

(57) L'invention concerne les supports de patient pour appareils de radiologie.

L'invention réside dans le fait que le déplacement vertical du plateau 10 est obtenu par un dispositif électromécanique constitué de bras 14 et 18 qui sont reliés entre eux et au plateau 10 par des articulations (12,16,21) motorisées et à rotations synchronisées. On obtient ainsi une hauteur minimale relativement faible, de l'ordre de 60 centimètres.

L'invention est applicable aux tables de radiologie.

FIG.1

EP 0 389 332 A1

## SUPPORT-PATIENT A GRAND DEBATTEMENT VERTICAL

L'invention concerne les supports de patient et, plus particulièrement, ceux qui sont mis en oeuvre dans les appareils de radiologie.

Un appareil de radiologie comprend une source de rayons X et un détecteur de rayonnement X que l'on déplace de manière coordonnée pour obtenir des images radiologiques d'un patient qui est habituellement allongé sur un support. Ce support est par exemple constitué d'un piédestal surmonté d'un plateau sur lequel est allongé le patient. Le piédestal et le plateau sont équipés de dispositifs électromécaniques pour déplacer le plateau suivant deux axes situés dans un plan horizontal et un troisième axe qui est vertical. Parfois, en plus, le piédestal est lui-même mobile sur des rails.

Pour des raisons de commodité d'examen du patient par le praticien et de facilité de passage de la source ou du détecteur sous le plateau, la hauteur maximale est d'environ 120 centimètres au-dessus du sol. Par ailleurs, la hauteur minimale qui est prévue par construction est d'environ un mètre. Cette hauteur minimale ne facilite pas le chargement du patient sur le plateau ou son déchargement car les lits roulants ont une hauteur plus faible.

Bien entendu, pour remédier à ce problème, il paraît suffire de modifier les dispositifs mécaniques existants pour obtenir une hauteur minimale plus faible, par exemple 60 centimètres. Une telle solution n'est pas possible car les mécanismes habituellement mis en oeuvre sur les tables ne sont pas du type rétractable ou repliable.

Le but de la présente invention est donc de réaliser un support-patient dont le débattement vertical est grand de manière à obtenir une hauteur minimale relativement faible, ce qui facilite le chargement ou le déchargement du patient.

L'invention se rapporte à un support patient caractérisé en ce qu'il comprend un plateau dont une extrémité est en porte-à-faux tandis que l'autre est fixée à une première articulation à axe de rotation horizontal, un premier bras à une extrémité duquel est fixée ladite première articulation et dont l'autre extrémité comporte une deuxième articulation à axe de rotation horizontal, un deuxième bras à une extrémité duquel est fixée ladite deuxième articulation et dont l'autre extrémité comporte une troisième articulation à axe de rotation horizontal, et un piédestal solidaire du sol sur lequel est fixée la troisième articulation, lesdites articulations étant motorisées et équipées de servomécanismes commandés par un microprocesseur de manière que le déplacement des bras entraîne un déplacement vertical du plateau, ce dernier restant parallèle au plan horizontal.

D'autres caractéristiques et avantages de la présente invention apparaîtront à la lecture de la description suivante d'un exemple particulier de réalisation, ladite description étant faite en relation avec les dessins joints dans lesquels :

- la figure 1 est une vue en perspective, partie en arraché, du support-patient selon la présente invention,

- la figure 2 est un schéma permettant de définir les relations à respecter pour déplacer le support, le plateau restant horizontal lors du déploiement du support-patient,

- la figure 3 est une vue de côté du support-patient complètement déployé verticalement,

- la figure 4 est une vue de côté du support-patient pour un déploiement intermédiaire, et

- la figure 5 est une vue de côté du support-patient pour un déploiement minimal.

Le support-patient selon l'invention comprend un plateau horizontal 10 qui est maintenu en porte-à-faux à une extrémité 11 sur une première articulation 12.

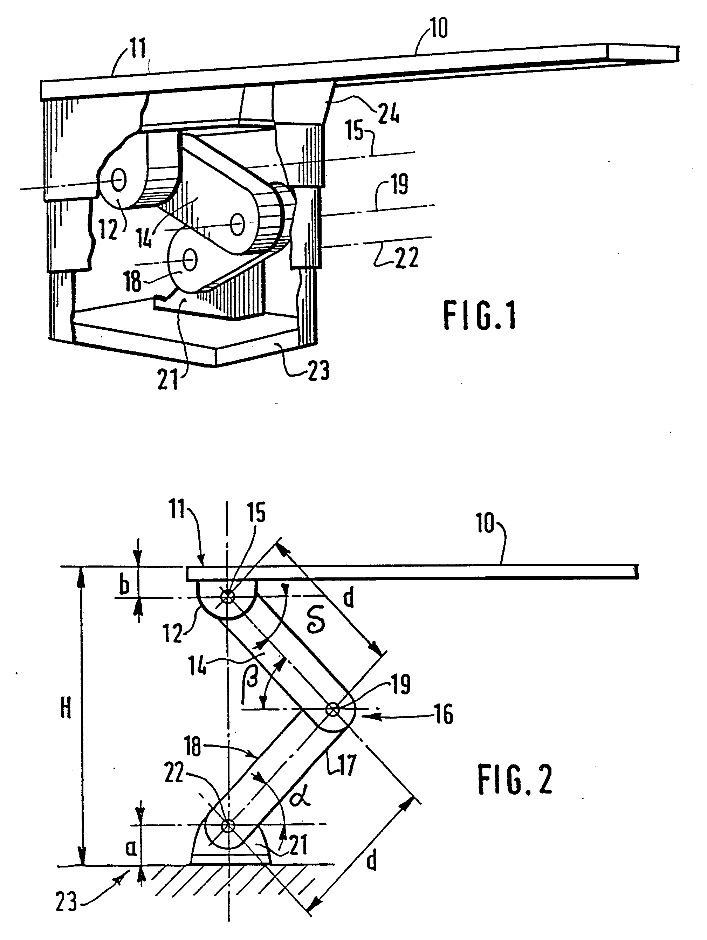

Cette articulation 12 est fixée à une extrémité d'un premier bras 14 et permet au plateau 10 de tourner autour d'un axe horizontal 15 (angle $\delta$ - figure 2) parallèle au plan du plateau 10 et perpendiculaire au plan de la figure 2. L'autre extrémité du bras 14 est reliée par une deuxième articulation 16 à une extrémité 17 d'un deuxième bras 18. Cette articulation fixée au bras 18 permet au bras 14 de tourner autour d'un axe horizontal 19 parallèle à l'axe 15 (angle $\beta$ - figure 2). Enfin, l'autre extrémité du bras 18 repose sur un piédestal 23, solidaire du sol 29, par l'intermédiaire d'une troisième articulation 21 qui permet au bras 18 de tourner autour d'un axe horizontal 22 (angle $\alpha$ - figure 2) parallèle aux axes 15 et 19.

Les articulations 12, 16 et 21 peuvent être motorisées sur chacun des axes et équipées chacune d'un servomécanisme commandé par un microprocesseur (non représenté). Dans une réalisation alternative, une seule articulation est motorisée et les mouvements angulaires des deux autres articulations sont synchronisés mécaniquement avec la première. Dans les deux cas, les mouvements angulaires des articulations doivent être tels que le déploiement ou le repliement des bras obéissent aux deux relations suivantes :

$\alpha = \beta = \delta$

$H = a + b + 2 d \sin \alpha$

- H étant la hauteur de la table par rapport au niveau du sol 29,

- a étant la hauteur de l'axe 22 par rapport au sol,

- b étant la distance entre l'axe 15 et le dessus du plateau 10

- d étant la longueur des bras 14 et 18.

La première relation correspond à des pivotements synchronisés des pivotements des bras et du plateau.

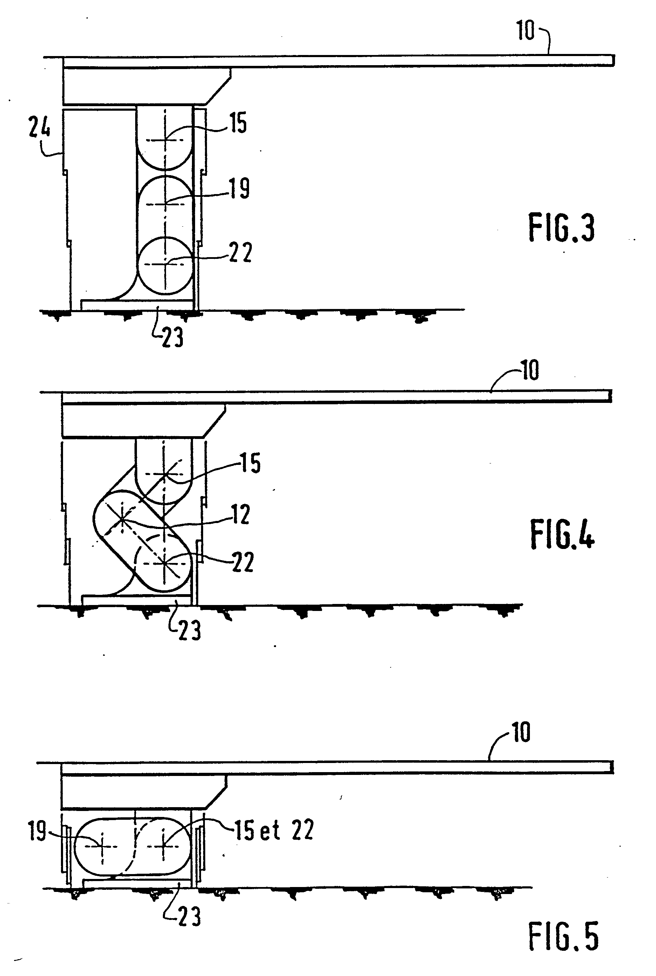

Afin de cacher l'ensemble des bras et articulations, le plateau 10 est muni, à l'aplomb des bras et articulations, d'une jupe 24 en trois parties qui s'emboîtent l'une dans l'autre lors du repliement. Lorsque le support-patient est complètement déployé (figure 3) les angles $\alpha$, $\beta$ et $\delta$ sont égaux à 90° et les trois axes 15, 19 et 22 sont alignés dans un même plan vertical perpendiculaire au plan de la figure 3. Par ailleurs, les trois parties de la jupe 24 sont également déployés.

La figure 4 représente une position intermédiaire en hauteur du support patient correspondant par exemple à $\alpha = \beta = \delta = 45°$. On remarquera que le mouvement des bras a lieu dans le sens inverse de celui prévu sur la figure 2 en cas de repliement. Les parties de la jupe se recouvrent partiellement.

Enfin, la figure 5 représente la position de repliement complet, les deux bras 14 et 18 étant parallèles l'un à l'autre et au plan horizontal et les trois axes 15, 19 et 22 étant dans un même plan horizontal. Les parties de la jupe se recouvrent complètement.

Afin que les bras 14 et 18 et les articulations 12, 16 et 22 puissent se replier l'un sur l'autre, il faut qu'ils soient disposés dans des plans verticaux différents et parallèles, ce que montre la vue en perspective de la figure 1.

L'invention a été décrite avec un dispositif comportant deux bras et trois articulations mais elle s'applique avec un nombre de bras et d'articulations plus élevé ou moins élevé.

Par ailleurs, les moyens pour obtenir les mouvements angulaires des articulations et leur synchronisation n'ont pas été décrits de manière détaillée car ils sont à la portée de l'homme de métier sans faire oeuvre d'invention.

## Revendications

1. Support-patient qui comprend un plateau (10) dont une extrémité est en porte-à-faux tandis que l'autre (11) est fixée à une première articulation (12) à axe de rotation horizontal (15), un premier bras (14) à une extrémité duquel est fixée ladite première articulation et dont l'autre extrémité comporte une deuxième articulation (16) à axe de rotation horizontal (19), un deuxième bras (18) à une extrémité duquel est fixée ladite deuxième articulation (16) et dont l'autre extrémité comporte une troisième articulation (21) à axe de rotation horizontal (22), et un piédestal (23) solidaire du sol sur lequel est fixée la troisième articulation (21), caractérisé en ce que lesdites articulations (12,16,21) comportent des moyens pour être animées de mouvements angulaires tels que les pivotements ($\alpha,\beta,\delta$) des bras (14,18) et du plateau (10) sont synchronisés tels que $\alpha = \beta = \delta$ et entraînent un déplacement vertical du plateau, ce dernier restant parallèle au plan horizontal.

2. Support patient selon la revendication 1 caractérisé en ce que les moyens d'animation des articulations comprennent au moins un moteur d'entraînement d'une articulation et des moyens mécaniques pour synchroniser les mouvements des deux autres articulations avec celui de l'articulation comportant le moteur d'entraînement.

3. Support patient selon la revendication 1 caractérisé en ce que les moyens d'animation des articulations (12,16,21) comprennent un moteur d'entraînement par articulation et des moyens pour synchroniser les mouvements desdits moteurs.

4. Support-patient selon l'une quelconque des revendications précédentes 1 à 3 caractérisé en ce que le nombre de bras est inférieur à deux et en ce que le nombre d'articulations est inférieur à trois.

5. Support-patient selon l'une quelconque des revendications précédentes 1 à 3, caractérisé en ce que le nombre de bras est supérieur à deux et en ce que le nombre d'articulations est supérieur à trois.

FIG.1

FIG.2

**FIG.3**

**FIG.4**

**FIG.5**

Office européen
des brevets

# RAPPORT DE RECHERCHE EUROPEENNE

Numero de la demande

EP    90 40 0660

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | EP-A-220501 (SIEMENS AG)<br>* figures *<br>* colonne 2, ligne 47 - colonne 3, ligne 36 * | 1 | A61B6/04 |
| A |  | 2, 3 | |
| A | US-A-3818516 (HOPPER ET AL)<br>* figures 1, 2 *<br>* colonne 3, ligne 5 - colonne 4, ligne 8 * | 1, 4 | |
| A | DE-B-2201921 (SIEMENS AG)<br>* figures 1-3 *<br>* colonne 2, ligne 53 - colonne 3, ligne 58 * | 1-3, 5 | |

DOMAINES TECHNIQUES
RECHERCHES (Int. Cl.5 )

A61B

Le présent rapport a été établi pour toutes les revendications

| Lieu de la recherche | Date d'achèvement de la recherche | Examinateur |
|---|---|---|
| LA HAYE | 04 JUILLET 1990 | CHEN A.H. |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
...................................................................................
& : membre de la même famille, document correspondant

EPO FORM 1503 03.82 (P0402)